# EUROPÄISCHE PATENTANMELDUNG

(11) **EP 3 246 009 A1**
(43) Veröffentlichungstag der Anmeldung: **22.11.2017**
(21) Anmeldenummer: 16170203.0
(22) Anmeldetag: 18.05.2016
(51) Int. Cl.: A61K 8/46, C11D 1/00, A61Q 5/02, A61Q 9/02, A61Q 19/10

(54) **WÄSSRIGE TENSID-ZUSAMMENSETZUNGEN**

(71) Anmelder: BASF SE, 67056 Ludwigshafen am Rhein (DE)
(72) Erfinder: Brunn, Claudia, 40597 Duesseldorf (DE); Behler, Ansgar, 46240 Bottrop (DE)
(74) Vertreter: BASF IP Association

(57) **Zusammenfassung**

Vorgeschlagen werden wässrige Tensid-Zusammensetzungen enthaltend
• ein oder mehrere alpha-Sulfofettsäuredisalze (A) der allgemeinen Formel (I),

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
• ein oder mehrere Sulfonate (B) die ausgewählt werden aus der Gruppe der Alkansulfonate der allgemeinen Formel (IIa), der alpha-Olefinsulfonate der allgemeinen Formel (IIb) und der Alkylbenzolsulfonate der allgemeinen Formel (IIc)

R²⁷-SO₃M¹⁴ (IIa)

R²⁸-SO₃M¹⁴ (IIb)

R²⁹-C₆H₄-SO₃M¹⁴ (IIc)

wobei der Rest R²⁷ einen linearen Alkylrest mit 8 bis 20 C-Atomen, der Rest R²⁸ einen linearen, einfach ungesättigten Alkenylrest mit 8 bis 20 C-Atomen und der Rest R²⁹ einen linearen oder verzweigten Alkylrest mit 8 bis 20 C-Atomen bedeutet und der Rest M¹⁴ ausgewählt wird aus der Gruppe H, Li, Na, K, Ca/2, Mg/2, Ammonium und Alkanolamine,
• Wasser,
wobei im Hinblick auf sogenannte Estersulfonate eine in den Patentansprüchen näher definierte Randbedingung einzuhalten ist und außerdem folgende Maßgaben gelten:
wobei folgende Maßgaben gelten:
• In Bezug auf die Verbindungen (A) gilt, dass der Anteil der Verbindungen (A), bei denen der Rest R¹ ein Alkenylrest ist, - bezogen auf die Gesamtmenge der Verbindungen (A) in den wäßrigen Tensid-Zusammensetzungen - bei 3 Gew.-% oder weniger liegt;
• in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 70 Gew.-% oder mehr liegt.

Diese Zusammensetzungen weisen ein gutes Schaumvermögen und eine angenehme Sensorik des Schaumes sowie eine gute Hautverträglichkeit auf und eignen sich für kosmetische Mittel sowie Wasch- und Reinigungsmittel.

## Beschreibung

### Gebiet der Erfindung

Die vorliegende Erfindung betrifft wässrige Tensid-Zusammensetzungen mit einem Gehalt an alpha-Sulfofettsäuredisalzen und speziellen Sulfonaten, die ausgewählt werden aus der Gruppe der Alkansulfonate, alpha-Olefinsulfonate und Alkylbenzolsulfonate.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel ein gutes Schaumvermögen und eine angenehme Sensorik des Schaumes erwünscht. Des Weiteren besteht ein Bedarf an anionischen Tensiden, die zumindest teilweise aus biogenen Quellen und speziell auch nachwachsenden Rohstoffen hergestellt werden können.

WO-A-92/15660 offenbart flüssige Reinigungsmittel mit einem Gehalt an sulfoölsauren Disalzen. Es wird offenbart, dass sulfoölsauren Disalze in der Lage sind, die Viskosität von Tensiden oder Tensidgemischen für Reinigungsmittel - insbesondere auf Basis von Fettalkylsulfaten, Fettalkylethersulfaten, Alkylpolyglucosiden und Fettsäuremonoethanolamiden - zu senken und zwar ebenso effektiv oder sogar besser wie durch Zusatz von Ethanol oder Hydrotropen (Seite 2, zweiter Absatz).

### Beschreibung der Erfindung

Die Aufgabe der vorliegenden Erfindung hat darin bestanden, wässrige Tensid-Zusammensetzungen bereitzustellen, die sich durch die im Folgenden genannten Eigenschaften auszeichnen:
- Gutes Schaumvermögen.
- Angenehme Sensorik des Schaumes.
- Gute Hautverträglichkeit.

Gegenstand der Erfindung sind zunächst wässrige Tensid-Zusammensetzungen enthaltend
- ein oder mehrere **alpha-Sulfofettsäuredisalze (A)** der allgemeinen Formel (I),

   R¹CH(SO₃M¹)COOM² (I),

   worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine,
- ein oder mehrere **Sulfonate (B)** die ausgewählt werden aus der Gruppe der Alkansulfonate der allgemeinen Formel (IIa), der alpha-Olefinsulfonate der allgemeinen Formel (IIb) und der Alkylbenzolsulfonate der allgemeinen Formel (IIc)

   R²⁷-SO₃M¹⁴ (IIa)

   R²⁸-SO₃M¹⁴ (IIb)

   R²⁹-C₆H₄-SO₃M¹⁴ (IIc)

   wobei der Rest R²⁷ einen linearen Alkylrest mit 8 bis 20 C-Atomen, der Rest R²⁸ einen linearen, einfach ungesättigten Alkenylrest mit 8 bis 20 C-Atomen und der Rest R²⁹ einen linearen oder verzweigten Alkylrest mit 8 bis 20 C-Atomen bedeutet und der Rest M¹⁴ ausgewählt wird aus der Gruppe H, Li, Na, K, Ca/2, Mg/2, Ammonium und Alkanolamine,
- **Wasser,**
wobei folgende Maßgaben gelten:
- In Bezug auf die Verbindungen (A) gilt, dass der Anteil der Verbindungen (A), bei denen der Rest R¹ ein Alkenylrest ist, - bezogen auf die Gesamtmenge der Verbindungen (A) in den wäßrigen Tensid-Zusammensetzungen - bei 3 Gew.-% oder weniger liegt;
- in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 70 Gew.-% oder mehr liegt;
- sofern die wässrigen Tensid-Zusammensetzungen ein oder mehrere Estersulfonate (E) der allgemeinen Formel (V),

   R²CH(SO₃M⁷)COOR³ (V)

   worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr - und insbesondere zu 90 Gew.-% oder mehr - vorliegen müssen.

### Zu den Verbindungen (A)

Die Verbindungen (A), die im Rahmen der vorliegenden Erfindung als **alpha-Sulfofettsäuredisalze** bezeichnet werden, sind für die erfindungsgemäßen wässrigen Tensid-Zusammensetzungen obligatorisch. Sie haben die oben angegebene Formel (I)

R¹CH(SO₃M¹)COOM² (I),

worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamine. Dabei gelten für die Verbindungen (A) die oben genannten Bedingungen:
- Der Anteil der Verbindungen (A), bei denen der Rest R¹ ein Alkenylrest ist, - bezogen auf die Gesamtmenge der Verbindungen (A) in den wäßrigen Tensid-Zusammensetzungen - liegt bei 3 Gew.-% oder weniger und
- der Anteil der Verbindungen (A), bei denen der Rest R¹ ein Decyl- oder ein Dodecylrest ist, - bezogen auf die Gesamtmenge der Verbindungen (A) - liegt bei 70 Gew.-% oder mehr.

Bezüglich der Reste M¹ und M² besonders bevorzugte Alkanolamine werden dabei ausgewählt aus der Gruppe Monoethanolamin, Diethanolamin, Triethanolamin und Mono-Isopropanolamin.

In einer bevorzugten Ausführungsform bedeutet der Rest R¹ in der Formel (I) einen gesättigten, linearen Alkylrest mit 10 bis 16 C-Atomen, wobei in Bezug auf die Verbindungen (A) gilt, dass der Anteil der Verbindungen (A), bei denen der Rest R¹ ein Decyl- und/oder ein Dodecylrest ist, - bezogen auf die Gesamtmenge der Verbindungen (A) - bei 90 Gew.-% oder mehr liegt.

Vorzugsweise werden die Reste M¹ und M² in der Formel (I) ausgewählt aus der Gruppe H (Wasserstoff) und Na (Natrium).

Die Verbindungen (A) können nach allen dem Fachmann einschlägig bekannten Methoden hergestellt werden. Eine besonders bevorzugte Methode der Herstellung ist dabei die Sulfierung der entsprechenden Carbonsäuren. Dabei setzt man die entsprechenden Carbonsäure und insbesondere die entsprechenden Fettsäuren mit gasförmigem Schwefeltrioxid um, wobei man das Schwefeltrioxid vorzugsweise in einer Menge einsetzt, dass das molare Verhältnis von SO₃ zu Fettsäure im Bereich von 1,0 : 1 bis 1,1 : 1 liegt. Die so erhaltenen Rohprodukte, die saure Sulfierprodukte darstellen, werden anschließend partiell oder vollständig neutralisiert, wobei eine vollständige Neutralisation mit wässriger NaOH bevorzugt ist. Gewünschtenfalls können auch Reinigungsschritte und/oder eine Bleiche (zur Einstellung der gewünschten hellen Farbe der Produkte) vorgenommen werden.

In einer besonders bevorzugten Ausführungsform werden die Verbindungen (A) in technischer Form eingesetzt. Dies bedeutet, dass man die entsprechenden Carbonsäuren, insbesondere native Fettsäure, mit gasförmigem Schwefeltrioxid sulfiert, wodurch nach partieller oder vollständiger Neutralisation der entstehenden sauren Sulfierprodukte ein Gemisch der Verbindungen (A), (C) und (D) resultiert. Durch entsprechende Einstellungen der Reaktionsparameter (insbesondere Mol-Verhältnis von Carbonsäure und Schwefeltrioxid sowie Reaktionstemperatur) lässt sich das Verhältnis der Verbindungen (A), (C) und (D) steuern. Die Verbindungen (C) und (D) sind unten im Kapitel "Bevorzugte Ausführungsformen" beschrieben.

Im Rahmen der vorliegenden Erfindung sind solche technischen Mischungen der alpha-Sulfofettsäuredisalze bevorzugt, die wie folgt zusammengesetzt sind:
- Der Gehalt an (A) liegt im Bereich von 60 bis 100 Gew.-%,
- der Gehalt an (C) liegt im Bereich von 0 bis 20 Gew.-%,
- der Gehalt an (D) liegt im Bereich von 0 bis 20 Gew.-%,
mit der Maßgabe, dass die Summe der Komponenten (A), (C) und (D) in dieser Mischung 100 Gew.-% beträgt.

### Zu den Verbindungen (B)

Die Verbindungen (B), die im Rahmen der vorliegenden Erfindung als Sulfonate bezeichnet werden, sind für die erfindungsgemäßen wässrigen Tensid-Zusammensetzungen obligatorisch. Die Sulfonate werden ausgewählt aus der Gruppe der Alkansulfonate der allgemeinen Formel (IIa), der alpha-Olefinsulfonate der allgemeinen Formel (IIb) und der Alkylbenzolsulfonate der allgemeinen Formel (IIc)

R²⁷-SO₃M¹⁴ (IIa)

R²⁸-SO₃M¹⁴ (IIb)

R²⁹-C₆H₄-SO₃M¹⁴ (IIc)

wobei der Rest R²⁷ einen linearen Alkylrest mit 8 bis 20 C-Atomen, der Rest R²⁸ einen linearen, einfach ungesättigten Alkenylrest mit 8 bis 20 C-Atomen und der Rest R²⁹ einen linearen oder verzweigten Alkylrest mit 8 bis 20 C-Atomen bedeutet und der Rest M¹⁴ ausgewählt wird aus der Gruppe H, Li, Na, K, Ca/2, Mg/2, Ammonium und Alkanolamine,

Die Verbindungen (B) können nach allen dem Fachmann einschlägig bekannten Methoden hergestellt werden. Die Alkansulfonate können beispielsweise durch Sulfoxidation oder durch Sulfochlorierung von unverzweigten Alkanen gewonnen werden. Die Alpha-Olefinsulfonate können beispielweise durch die Sulfonierung von Alpha-Olefinen mit gasförmigem Schwefeltrioxid auf einem Fallfilmreaktor und anschließender Neutralisation hergestellt werden. Die Alkylbenzolsulfonate können beispielsweise durch Umsetzung von Alkylbenzol mit einem geeigneten Sulfonierungsmittel (Oleum, Schwefelsäure, SO₃) gewonnen werden.

In einer bevorzugten Ausführungsform bedeutet der Rest R²⁷ in der Formel (IIa) einen linearen, sekundären Alkylrest mit 12 bis 18 C-Atomen.

In einer bevorzugten Ausführungsform bedeutet der Rest R²⁸ in der Formel (IIb) einen linearen, einfach ungesättigten Alkenylrest mit 12 bis 18 C-Atomen, wobei sich die C=C-Doppelbindung am zweiten oder dritten C-Atom befindet. In einer bevorzugten Ausführungsform handelt es sich bei Verbindung (IIb) um Sodium-C14-16-Olefin-Sulfonate (INCI-Bezeichnung).

In einer bevorzugten Ausführungsform bedeutet der Rest R²⁹ in der Formel (IIc) einen linearen Alkylrest mit 12 bis 18 C-Atomen, wobei die Sulfonatgruppe sich an der ersten und der Alkylrest sich an der vierten Position des Benzolrings befindet und der Benzolring an ein beliebiges C-Atom der Alkylkette gebunden sein kann (Isomere). In einer bevorzugten Ausführungsform handelt es sich bei Verbindung (IIc) um Sodium-Dodecylbenzensulfonate (INCI-Bezeichnung).

Vorzugsweise wird der Rest M¹⁴ in der Formel (IIa), (IIb) und (IIc) ausgewählt aus der Gruppe H (Wasserstoff) und Na (Natrium).

### Bevorzugte Ausführungsformen

In einer Ausführungsform enthalten die erfindungsgemäßen wässrigen Tensid-Zusammensetzungen neben den Verbindungen (A), (B) und Wasser zusätzlich ein oder mehrere **Verbindungen** (C) der allgemeinen Formel (III)

R⁴COOM⁵ (III)

In der Formel (III) bedeutet der Rest R⁴ einen linearen oder verzweigten Alkyl- oder Alkenylrest mit 7 bis 19 C-Atomen und der Reste M⁵ wird ausgewählt aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

In einer Ausführungsform enthalten die erfindungsgemäßen wässrigen Tensid-Zusammensetzungen neben den Verbindungen (A), (B) und Wasser zusätzlich ein oder mehrere **anorganische Salze der Schwefelsäure (D)** der allgemeinen Formel (IV)

(M⁶)₂SO₄ (IV)

wobei M⁶ ausgewählt wird aus der Gruppe Li, Na, K, Ca/2, Mg/2, Ammonium und Alkanolamin. Besonders bevorzugte Alkanolamine sind dabei Monoethanolamin, Diethanolamin, Triethanolamin und Mono-Isopropanolamin.

In einer bevorzugten Ausführungsform enthalten die erfindungsgemäßen wässrigen Tensid-Zusammensetzungen die Verbindungen (A), (B), (C) und (D). Dabei ist es besonders bevorzugt, wenn die Reste M¹ und M² der Verbindungen (A), der Rest M¹⁴ der Verbindungen (B), der Rest M⁵ der Verbindungen (C) und der Rest M⁶ der Verbindungen (D) ausgewählt werden aus der Gruppe H( Wasserstoff) und Na (Natrium).

In einer Ausführungsform enthalten die erfindungsgemäßen wässrigen Tensid-Zusammensetzungen neben den Verbindungen (A), (B) und Wasser zusätzlich ein oder mehrere **Verbindungen (F)** der allgemeinen Formel (VI)

R⁶CH₂-CO-CHR⁷(SO₃M⁸) (VI),

worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (F) werden im Rahmen der vorliegenden Erfindung als Mono-Sulfo-Ketone bezeichnet.

In einer bevorzugten Ausführungsform bedeuten die Reste R⁶ und R⁷ in der Formel (VI) - unabhängig voneinander - einen gesättigten, linearen Rest mit 10 bis 16 C-Atomen, wobei in Bezug auf die Verbindungen (F) gilt, dass der Anteil der Verbindungen (F), bei denen die Reste R⁶ und R⁷ einen Decyl- und/oder ein Dodecylrest bedeuten, - bezogen auf die Gesamtmenge der Verbindungen (F) - bei 70 Gew.-% oder mehr und vorzugsweise bei 90 Gew.-% oder mehr liegt. Vorzugsweise wird der Rest M⁸ in der Formel (VI) ausgewählt aus der Gruppe H und Na.

In einer Ausführungsform enthalten die erfindungsgemäßen wässrigen Tensid-Zusammensetzungen neben den Verbindungen (A), (B) und Wasser zusätzlich ein oder mehrere **Verbindungen (G)** der allgemeinen Formel (VII)

(SO₃M⁹)R⁸CH-CO-CHR⁹(SO₃M¹⁰) (VII),

worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine. Besonders bevorzugte Alkanolamine sind dabei Monoethanolamin, Diethanolamin, Triethanolamin und Mono-Isopropanolamin.

Die Verbindungen (G) werden im Rahmen der vorliegenden Erfindung als Di-Sulfo-Ketone bezeichnet.

In einer bevorzugten Ausführungsform bedeuten die Reste R⁸ und R⁹ in der Formel (VII) - unabhängig voneinander - einen gesättigten, linearen Rest mit 10 bis 16 C-Atomen, wobei in Bezug auf die Verbindungen (G) gilt, dass der Anteil der Verbindungen (G), bei denen die Reste R⁸ und R⁹ einen Decyl- und/oder ein Dodecylrest bedeuten, - bezogen auf die Gesamtmenge der Verbindungen (G) - bei 70 Gew.-% oder mehr und vorzugsweise bei 90 Gew.-% oder mehr liegt. Vorzugsweise werden die Reste M⁹ und M¹⁰ in der Formel (VII) ausgewählt aus der Gruppe H und Na.

Die Herstellung der Verbindungen (F) und (G) unterliegt keinen besonderen Einschränkungen und sie können nach allen dem Fachmann bekannten Methoden hergestellt werden.

In einer Ausführungsform werden die Verbindung (F) und (G) durch Sulfonierung entsprechender Ketone mit gasförmigem Schwefeltrioxid hergestellt, wie in der deutschen Offenlegungsschrift DE-A-42,20,580 beschrieben.

In einer anderen Ausführungsform geht man zur Herstellung der Verbindungen (F) und (G) von Fettsäuren aus. Dabei führt man die Sulfierung von flüssigen Fettsäuren mit gasförmigem Schwefeltrioxid so durch, dass dabei neben Disalzen (A) auch die Verbindungen (F) und (G) entstehen, was dadurch realisiert werden kann, dass man die Sulfierung wie folgt durchführt: Das Verhältnis der Rohstoffe Fettsäure, die auch in Form von Gemischen von Fettsäuren unterschiedlicher Kettenlänge eingesetzt werden können, und Schwefeltrioxid wird so eingestellt, dass man 1,0 bis 1,5 mol und insbesondere 1,0 bis 1,25 mol SO₃ pro mol Fettsäure(n) einsetzt. Die Fettsäuren werden dabei mit einer Vorlagetemperatur im Bereich von 70 bis 100 °C in den Reaktor eingebracht. Nach der Sulfierung wird das erhaltene flüssige Sulfierprodukt in einer temperierten Nachreaktionsschlange für 5 bis 20 Minuten auf dieser Temperatur gehalten und gealtert. Anschließend erfolgt die Neutralisation mit einer wässrigen Base, vorzugsweise Natriumhydroxid, in der Regel bei einem pH-Wert im Bereich von von 5 bis 10, insbesondere von 5 bis 7. Im Anschluss kann eine saure Bleiche - der pH wird hierbei auf einen Wert von 7 oder weniger eingestellt - mit Wasserstoffperoxid durchgeführt werden.

In einer Ausführungsform enthalten die erfindungsgemäßen wässrigen Tensid-Zusammensetzungen die Verbindungen (A), (B) und (F). Dabei ist es besonders bevorzugt, wenn die Reste M¹ und M² der Verbindungen (A) und der Rest M¹⁴ der Verbindungen (B) ausgewählt werden aus der Gruppe H (Wasserstoff) und Na (Natrium). Dabei gilt die Maßgabe, dass die Menge der Verbindungen (A) größer sein muss als die Menge der Verbindungen (F).

In einer Ausführungsform enthalten die erfindungsgemäßen wässrigen Tensid-Zusammensetzungen die Verbindungen (A), (B) und (G). Dabei ist es besonders bevorzugt, wenn die Reste M¹ und M² der Verbindungen (A) und der Rest M¹⁴ der Verbindungen (B) ausgewählt werden aus der Gruppe H und Na. Dabei gilt die Maßgabe, dass die Menge der Verbindungen (A) größer sein muss als die Menge der Verbindungen (G).

In einer Ausführungsform enthalten die erfindungsgemäßen wässrigen Tensid-Zusammensetzungen die Verbindungen (A), (B), (F) und (G). Dabei ist es besonders bevorzugt, wenn die Reste M¹ und M² der Verbindungen (A) und der Rest M¹⁴ der Verbindungen (B) ausgewählt werden aus der Gruppe H und Na. Dabei gilt die Maßgabe, dass die Menge der Verbindungen (A) größer sein muss als die Summe der Menge der Verbindungen (F) und (G).

In einer Ausführungsform enthalten die erfindungsgemäßen wässrigen Tensid-Zusammensetzungen die Verbindungen (A), (B), (C), (D) und (F). Dabei ist es besonders bevorzugt, wenn die Reste M¹ und M² der Verbindungen (A), der Rest M¹⁴ der Verbindungen (B), der Rest M⁵ der Verbindungen (C) und der Rest M⁶ der Verbindungen (D) ausgewählt werden aus der Gruppe H und Na. Dabei gilt die Maßgabe, dass die Menge der Verbindungen (A) größer sein muss als die Menge der Verbindungen (F).

In einer Ausführungsform enthalten die erfindungsgemäßen wässrigen Tensid-Zusammensetzungen die Verbindungen (A), (B), (C), (D) und (G). Dabei ist es besonders bevorzugt, wenn die Reste M¹ und M² der Verbindungen (A), der Rest M¹⁴ der Verbindungen (B), der Rest M⁵ der Verbindungen (C) und der Rest M⁶ der Verbindungen (D) ausgewählt werden aus der Gruppe H und Na. Dabei gilt die Maßgabe, dass die Menge der Verbindungen (A) größer sein muss als die Menge der Verbindungen (G).

In einer Ausführungsform enthalten die erfindungsgemäßen wässrigen Tensid-Zusammenetzungen die Verbindungen (A), (B), (C), (D), (F) und (G). Dabei ist es besonders bevorzugt, wenn die Reste M¹ und M² der Verbindungen (A), der Rest M¹⁴ der Verbindungen (B), der Rest M⁵ der Verbindungen (C) und der Rest M⁶ der Verbindungen (D) ausgewählt werden aus der Gruppe H und Na. Dabei gilt die Maßgabe, dass die Menge der Verbindungen (A) größer sein muss als die Summe der Menge der Verbindungen (F) und (G).

Gewünschtenfalls können die erfindungsgemäßen wässrigen Tensid-Zusammensetzungen zusätzlich ein oder mehrere weitere Tenside enthalten, die strukturell nicht zu den oben genannten Verbindungen (A), (B), (D), (E), (F) oder (G) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

Im Hinblick auf Reinigungsmittel sind dabei insbesondere Mittel mit niedrigem pH-Wert zur Reinigung harter Oberflächen bevorzugt, wie Bad- und WC-Reiniger und dergleichen, sowie für Reinigungs- und/oder Duftgele zur Anwendung in sanitären Einrichtungen.

## Patentansprüche

1. Wässrige Tensid-Zusammensetzungen enthaltend
• ein oder mehrere **alpha-Sulfofettsäuredisalze (A)** der allgemeinen Formel (I),
R¹CH(SO₃M¹)COOM² (I)
worin der Rest R¹ einen linearen oder verzweigten Alkyl- oder Alkenylrest mit 6 bis 18 C-Atomen bedeutet und die Reste M¹ und M² - unabhängig voneinander - ausgewählt werden aus der Gruppe H, Li, Na, K, Ca/2, Mg/2, Ammonium und Alkanolamin,
• ein oder mehrere **Sulfonate (B)** die ausgewählt werden aus der Gruppe der Alkansulfonate der allgemeinen Formel (IIa), der alpha-Olefinsulfonate der allgemeinen Formel (IIb) und der Alkylbenzolsulfonate der allgemeinen Formel (IIc)
R²⁷-SO₃M¹⁴ (IIa)
R²⁸-SO₃M¹⁴ (IIb)
R²⁹-C₆H₄-SO₃M¹⁴ (IIc)
wobei der Rest R²⁷ einen linearen Alkylrest mit 8 bis 20 C-Atomen, der Rest R²⁸ einen linearen, einfach ungesättigten Alkenylrest mit 8 bis 20 C-Atomen und der Rest R²⁹ einen linearen oder verzweigten Alkylrest mit 8 bis 20 C-Atomen bedeutet und der Rest M¹⁴ ausgewählt wird aus der Gruppe H, Li, Na, K, Ca/2, Mg/2, Ammonium und Alkanolamine,
• **Wasser,**
wobei folgende Maßgaben gelten:
• In Bezug auf die Verbindungen (A) gilt, dass der Anteil der Verbindungen (A), bei denen der Rest R¹ ein Alkenylrest ist, - bezogen auf die Gesamtmenge der Verbindungen (A) in den wäßrigen Tensid-Zusammensetzungen - bei 3 Gew.-% oder weniger liegt;
• in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 70 Gew.-% oder mehr liegt;
• sofern die wässrigen Tensid-Zusammensetzungen ein oder mehrere Estersulfonate (E) der allgemeinen Formel (V),
R²CH(SO₃M⁷)COOR³ (V)
worin der Rest R² einen linearen oder verzweigten Alkyl- oder Alkenylrest mit 6 bis 18 C-Atomen bedeutet und der Rest R³ einen linearen oder verzweigten Alkyl- oder Alkenylrest mit 1 bis 20 C-Atomen bedeutet, wobei der Rest R³ logischerweise erst ab 3 C-Atomen ein Alkenylrest sein oder verzweigt sein kann, und der Rest M⁷ ausgewählt wird aus der Gruppe Li, Na, K, Ca/2, Mg/2, Ammonium und Alkanolamine, enthält, gilt, dass die Verbindungen (A) - bezogen auf die Gesamtheit der Verbindungen (A) und (E) - zu 50 Gew.-% oder mehr vorliegen müssen.

2. Zusammensetzungen nach Anspruch 1, wobei der Rest R¹ in der Formel (I) einen gesättigten, linearen Alkylrest mit 10 bis 16 C-Atomen bedeutet, wobei in Bezug auf die Verbindungen (A) gilt, dass der Anteil der Verbindungen (A), bei denen der Rest R¹ ein Decyl- oder ein Dodecylrest ist, - bezogen auf die Gesamtmenge der Verbindungen (A) - bei 90 Gew.-% oder mehr liegt.

3. Zusammensetzungen nach Anspruch 1 oder 2, wobei die Reste M¹ und M² ausgewählt werden aus der Gruppe H (Wasserstoff) und Na (Natrium).

4. Zusammensetzungen nach einem der Ansprüche 1 bis 3, wobei die Zusammensetzungen zusätzlich ein oder mehrere **Verbindungen (C)** der allgemeinen Formel (III)
R⁴COOM⁵ (III)
enthalten, worin der Rest R⁴ einen linearen oder verzweigten Alkyl- oder Alkenylrest mit 7 bis 19 C-Atomen bedeutet und der Reste M⁵ ausgewählt wird aus der Gruppe H, Li, Na, K, Ca/2, Mg/2, Ammonium und Alkanolamin.

5. Zusammensetzungen nach einem der Ansprüche 1 bis 4, wobei die Zusammensetzungen zusätzlich ein oder mehrere **anorganische Salze der Schwefelsäure (D)** der allgemeinen Formel (IV)
(M⁶)₂SO₄ (IV)
enthalten, wobei M⁶ ausgewählt wird aus der Gruppe Li, Na, K, Ca/2, Mg/2, Ammonium und Alkanolamin.

6. Zusammensetzungen nach einem der Ansprüche 1 bis 5, wobei die Zusammensetzungen zusätzlich ein oder mehrere **Mono-Sulfo-Ketone (F)** der allgemeinen Formel (VI)
R⁶CH₂-CO-CHR⁷(SO₃M⁸) (VI),
worin die Reste R⁶ und R⁷ - unabhängig voneinander - einen linearen oder verzweigten Alkylrest mit 6 bis 18 C-Atomen bedeuten und der Rest M⁸ ausgewählt wird aus der Gruppe H, Li, Na, K, Ca/2, Mg/2, Ammonium und Alkanolamine.

7. Zusammensetzungen nach einem der Ansprüche 1 bis 6, wobei die Zusammensetzungen zusätzlich ein oder mehrere **Di-Sulfo-Ketone (G)** der allgemeinen Formel (VII)
(SO₃M⁹)R⁸CH-CO-CHR⁹(SO₃M¹⁰) (VII),
worin die Reste R⁸ und R⁹ - unabhängig voneinander - einen linearen oder verzweigten Alkylrest mit 6 bis 18 C-Atomen bedeuten und die Reste M⁹ und M¹⁰ - unabhängig voneinander - ausgewählt werden aus der Gruppe H, Li, Na, K, Ca/2, Mg/2, Ammonium und Alkanolamine.

8. Verwendung der Zusammensetzungen nach einem der Ansprüche 1 bis 7 für kosmetische Mittel sowie Wasch- und Reinigungsmittel.

9. Verwendung der Zusammensetzungen nach einem der Ansprüche 1 bis 7 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

10. Verwendung der Zusammensetzungen nach einem der Ansprüche 1 bis 7 für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.
